# FASCICULE DE BREVET EUROPEEN

(11) **EP 0 852 154 B1**
(45) Date de publication et mention de la délivrance du brevet: **07.07.2004**
(21) Numéro de dépôt: 98400017.4
(22) Date de dépôt: 07.01.1998
(51) Int. Cl.: A61N 1/37, H03H 11/04

(54) **Dispositif de filtrage de signaux d'activité cardiaque.**
Vorrichtung zur Filterung von Signalen der Herztätigkeit
Device for filtering cardiac activity signals

(30) Priorité: 07.01.1997 FR 9700065
(43) Date de publication de la demande: 08.07.1998
(73) Titulaire: ELA MEDICAL (Société anonyme), 92541 Montrouge (FR)
(72) Inventeur: Legay, Thierry, 91640 Fontenay Les Briis (FR); Pons, Pascal, 38920 Crolles (FR); Garcia, Luc, 38330 Saint Ismier (FR)
(74) Mandataire: Dupuis-Latour, Dominique

(56) Documents cités:
- EP-A- 0 605 264
- PATENT ABSTRACTS OF JAPAN vol. 004, no. 137 (E-027), 25 septembre 1980 & JP 55 091220 A (PIONEER ELECTRONIC CORP), 10 juillet 1980,
- PATENT ABSTRACTS OF JAPAN vol. 095, no. 002, 31 mars 1995 & JP 06 325308 A (HITACHI LTD), 25 novembre 1994,

## Description

La présente invention concerne le traitement des signaux recueillis par les dispositifs médicaux, notamment par les "dispositifs médicaux implantables actifs" tels que définis par la Directive 90/385/CEE du 20 juin 1990 du Conseil des communautés européennes, par exemple les stimulateurs cardiaques, les défibrillateurs et/ou cardioverteurs, les appareils neurologiques, les pompes de diffusion de substances médicales et les implants cochléaires.

L'invention sera principalement décrite dans le cas d'un stimulateur cardiaque, mais il ne s'agit là que d'un exemple de mise en oeuvre de l'invention, qui est applicable de façon beaucoup plus générale à une très grande variété de dispositifs médicaux actifs, implantables ou même non implantables, par exemple portés par le patient.

Dans ces appareils, l'activité cardiaque est recueillie aux bornes d'électrodes et le signal obtenu est appliqué à un module d'amplification et de filtrage. L'amplificateur est généralement prévu pour recevoir des signaux de l'ordre du millivolt dans une bande de fréquence s'étendant sur une plage allant typiquement de 1 Hz à 80 Hz.

Mais les appareils les plus récents obligent à repousser ces limites.

En effet, par exemple, les stimulateurs du type VDD présentent des sensibilités beaucoup plus faibles, de l'ordre de 0,1 mV, car ils utilisent dans l'oreillette une électrode flottante pour le recueil des ondes P et, pour capter ces signaux, il est nécessaire d'accroître les gains des amplificateurs.

Par ailleurs, les stimulateurs actuels possèdent des fonctions dites "Holter", c'est-à-dire de mémorisation et d'analyse de l'activité cardiaque sur une très longue période, typiquement sur plusieurs heures. L'analyse du signal endocavitaire qui est opérée à cet effet nécessite une bande passante dont la fréquence minimale est beaucoup plus basse, typiquement 0,1 Hz, pour pouvoir par exemple analyser le segment ST du signal cardiaque.

Ces perfectionnements entraînent l'apparition de problèmes nouveaux, notamment du fait de la bande passante beaucoup plus large dans le domaine des basses fréquences.

Tout d'abord, indépendamment de sa sensibilité, l'amplificateur d'entrée doit pouvoir supporter l'amplitude élevée de l'impulsion de stimulation susceptible d'être appliquée par le dispositif, impulsion dont l'amplitude peut atteindre 10 V, et ensuite récupérer le plus rapidement possible une capacité d'écoute (sensibilité) de signaux de l'ordre du millivolt.

Pour pouvoir supporter la tension élevée de l'impulsion de stimulation, on prévoit toujours une période de "blanking" (déconnexion) des circuits d'entrée au moment où l'impulsion est délivrée. Toutefois, à la fin du blanking, au moment où l'amplificateur d'entrée est à nouveau commuté en écoute, une saturation importante de l'étage d'èntrée peut encore se produire car le potentiel de l'interface coeur/électrode n'est pas encore revenu à sa valeur de repos.

Ce problème est en outre aggravé par le fait que le temps de récupération de l'amplificateur est d'autant plus long que la fréquence de coupure du filtre passe-haut de l'étage d'entrée est plus faible : ainsi, pour un filtre passe-haut à coupure à 0,1 Hz, le temps de récupération de l'amplificateur est de l'ordre de 10 s, ce qui est totalement incompatible avec le besoin que l'on a de répondre de façon rapide à la succession des signaux cardiaques.

Il est certes possible d'appliquer la technique dite de "pré- ou post-charge", consistant à délivrer des charges électriques avant ou après l'impulsion de stimulation pour compenser l'allongement du temps de récupération. Toutefois, cette méthode est fortement consommatrice d'énergie et ne saurait être appliquée de façon permanente sans diminuer notablement la durée de vie du dispositif implanté.

Un autre problème réside dans la taille des composants capacitifs et résistifs, qui est d'autant plus élevée qu'est basse la fréquence de coupure du filtre passe-haut de l'étage d'entrée. On voit ainsi que plus l'on élargit la fenêtre d'écoute dans le domaine des basses fréquences, plus il est nécessaire de disposer de composants de grande taille, ce qui est incompatible avec les impératifs de miniaturisation des circuits des appareils implantables.

L'un des buts de l'invention est de pallier ces inconvénients, en proposant un dispositif qui permette d'élargir notablement la bande d'analyse spectrale dans le domaine des basses fréquences, sans pour autant requérir des composants de grande taille ni, surtout, affecter significativement le temps de récupération de l'amplificateur d'entrée du stimulateur.

Comme on le comprendra, ce problème n'est pas propre aux seuls stimulateurs cardiaques, et se pose chaque fois que l'on est en présence de signaux recueillis par des capteurs tels que capteur d'activité, de respiration, etc. dont on souhaite pouvoir exploiter ultérieurement la composante à très basse fréquence tout en ayant pour le traitement classique du signal des temps de réponse réduits.

Essentiellement, l'invention propose de choisir une fréquence de coupure du filtre passe-haut de l'étage d'entrée du stimulateur plus élevée que les valeurs habituelles (par exemple 4 Hz au lieu de 1 Hz) et de prévoir un étage de compensation, de préférence à filtrage numérique, qui compense l'atténuation due au filtre d'entrée.

L'étage de compensation peut n'être utilisé que lorsque l'on désire exploiter les composantes à très basse fréquence du signal. Il peut être soit intégré à la prothèse implantable (pour permettre par exemple une analyse en temps réel du signal cardiaque) soit incorporé par exemple à un programmateur ou autre appareil externe destiné à coopérer avec la prothèse implantée pour traiter les signaux recueillis.

Plus précisément, le dispositif de l'invention est un dispositif de filtrage qui est du type, connu, par exemple du document EP-A-0 605 264, recevant en entrée des signaux de moyens de recueil de données physiologiques, et délivrant en sortie, à des moyens de traitement de ces données, des signaux s'étendant, dans le domaine fréquentiel, sur une bande spectrale élargie, les moyens de recueil comprenant un premier filtre passe-haut réduisant l'étendue de la bande spectrale du signal reçu en entrée. Selon l'invention, il comprend un étage de compensation comportant un dispositif d'accentuation de caractéristique fréquentielle inversée par rapport à celle du premier filtre passe-haut, la fréquence de coupure du premier filtre passe-haut étant choisie supérieure à la fréquence inférieure de la bande spectrale élargie.

Selon un certain nombre de caractéristiques avantageuses :
- le dispositif comprend en outre un second filtre passe-haut, dont la caractéristique présente une fréquence de coupure correspondant à la fréquence inférieure de la bande spectrale élargie ;
- le dispositif d'accentuation et le premier filtre passe-haut ont même fréquence de coupure et des fonctions de transfert inverses ;
- l'ordre de la fonction de transfert du second filtre passe-haut est supérieur à l'ordre de la fonction de transfert du premier filtre passe-haut ;
- la fréquence inférieure de la bande spectrale élargie est de l'ordre de 0,1 Hz et la fréquence de coupure du premier filtre passe-haut est de l'ordre de 4 Hz.

L'invention est également applicable, de façon symétrique, à l'élargissement de la bande des fréquences vers le haut.

Ceci permet, notamment, de restreindre vers le haut la bande passante aux étages d'amplification et de numérisation en limitant corrélativement l'influence des signaux externes haute fréquence pouvant engendrer des saturations des étages d'amplification, mais en restaurant ultérieurement le signal utile dans toute la largeur de la bande spectrale pour permettre une analyse complète.

Dans cette dernière forme de mise en oeuvre, les moyens de recueil comprennent un premier filtre passe-bas réduisant l'étendue de la bande spectrale du signal reçu en entrée et, selon l'invention, le dispositif comprend un étage de compensation comportant un dispositif d'accentuation de caractéristique fréquentielle inversée par rapport à celle du premier filtre passe-bas, la fréquence de coupure du premier filtre passe-bas étant choisie inférieure à la fréquence inférieure de la bande spectrale élargie.

Dans ce cas, selon un certain nombre de caractéristiques avantageuses :
- le dispositif comprend en outre un second filtre passe-bas, dont la caractéristique présente une fréquence de coupure correspondant à la fréquence supérieure de la bande spectrale élargie ;
- le dispositif d'accentuation et le premier filtre passe-bas ont même fréquence de coupure et des fonctions de transfert inverses ;
- l'ordre de la fonction de transfert du second filtre passe-bas est supérieur à l'ordre de la fonction de transfert du premier filtre passe-bas.

Le dispositif de filtrage de l'invention, dans l'une ou l'autre de ses formes de réalisation, peut être incorporé à un dispositif médical implantable actif, à un programmateur externe prévu pour coopérer avec un dispositif médical actif implanté, ou bien encore à un boîtier d'interfaçage à un tel programmateur.

D'autres caractéristiques de l'invention apparaîtront à la lecture de la description d'un exemple de mise en oeuvre ci-dessous, en référence aux dessins annexés.
La figure 1 est un schéma par blocs de la chaîne de recueil et de traitement du signal cardiaque.
La figure 2 est un diagramme de Bode montrant la caractéristique d'atténuation du filtre de l'étage d'entrée du dispositif implanté.
La figure 3 est un diagramme de Bode montrant, respectivement. la caractéristique du filtre de l'étage d'entrée et les caractéristiques des filtres selon l'invention.
La figure 4 est un diagramme de Bode montrant la caractéristique de réponse spectrale résultante.
Les figures 5, 6 et 7 sont des chronogrammes montrant l'allure du signal cardiaque endocavitaire, respectivement tel que recueilli, filtré par l'étage d'entrée du stimulateur et reconstruit après application de la compensation selon l'invention.
Les figures 8, 9 et 10, symétriques des figures 2, 3 et 4, correspondent au cas où l'on souhaite élargir la bande spectrale dans le domaine des hautes fréquences.

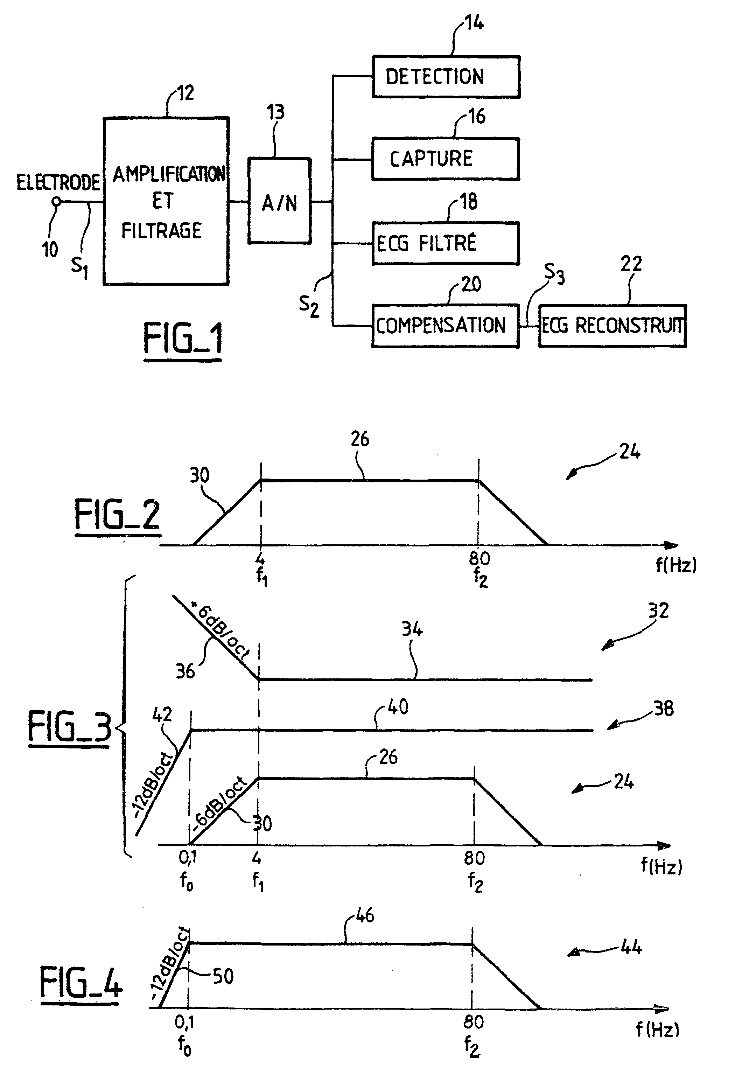

La figure 1 est un schéma par blocs d'un dispositif implantable actif, comprenant une électrode 10 recueillant un signal cardiaque endocavitaire S₁, qui est appliqué à un circuit 12 d'amplification et de filtrage puis à un convertisseur analogique/numérique 13. Ce convertisseur doit avoir la résolution nécessaire pour coder l'information utile du signal S₁ après qu'elle ait été atténuée par le bloc 12. Le signal amplifié, filtré et numérisé S₂ alimente une pluralité de modules, à savoir un module de détection 14, un module de capture 16 et un circuit 18 d'analyse de l'électrocardiogramme (ECG) filtré par le circuit 12.

Ces modules peuvent être des circuits spécifiques ou, en variante, des blocs logiciels opérant sur un signal préalablement échantillonné et numérisé au sein du bloc 12.

Comme on l'a indiqué au début de la description, les circuits actuels, tels que le circuit 18, d'analyse de l'ECG filtré opèrent sur une bande spectrale s'étendant typiquement de 1 Hz à 80 Hz, ces deux fréquences correspondant à des fréquences de coupure passe-haut et passe-bas, respectivement, du filtrage opéré dans le bloc 12.

Comme on l'a indiqué au début de la présente description, les circuits des stimulateurs actuels ne sont cependant pas adaptés aux performances des appareils les plus récents.

En effet, pour réaliser des fonctions Holter, il est nécessaire de pouvoir analyser des ECG dans une fenêtre d'écoute débutant à 0,1 Hz. Mais si l'on choisit pour le filtre passe-haut d'entrée une fréquence de coupure beaucoup plus faible, le temps de récupération de l'amplificateur devient prohibitif, ce qui crée une incompatibilité avec la nécessité de répondre de façon rapide à la succession des signaux cardiaques. En outre, un filtre passe-haut à fréquence de coupure très basse requiert des composants capacitifs et résistifs de taille importante, qui peuvent ne pas être intégrables dans un dispositif implantable.

Pour remédier à cette difficulté, l'invention propose de relever la fréquence de coupure du filtre passe-haut du bloc 12, par exemple - mais de façon bien entendu non limitative - de 1 à 4 Hz, et d'appliquer au signal S₂ une compensation inverse correspondante (bloc 20), pour délivrer un signal S₃, ou "signal reconstruit" s'étendant sur une fenêtre d'écoute très étendue dans le domaine des basses fréquences, typiquement jusqu'à 0,1 Hz - cette valeur n'étant bien entendu pas limitative. Le signal S₃ pourra être analysé par un module approprié (bloc 22), assurant par exemple une analyse du segment ST qui requiert une bande passante du signal ECG débutant en fréquence à 0,1 Hz.

Dans une première forme de mise en oeuvre, les modules 20 et 22 sont incorporés au dispositif implanté, de manière à permettre une étude en temps réel, et par le dispositif lui-même, du tracé endocavitaire.

Dans une autre forme de mise en oeuvre, les modules 20 et 22 sont situés dans un programmateur externe, par exemple sous forme de routines spécifique d'un logiciel d'analyse de signal cardiaque, le signal endocavitaire numérisé étant alors transmis au programmateur par des moyens de télémétrie.

Dans l'un et l'autre cas, le dispositif de compensation 20, tout comme le filtre passe-haut du bloc 12, peuvent être réalisés sous forme analogique, par des circuits de filtrage spécifiques. Mais il peuvent aussi, avantageusement, être réalisé de façon numérique, sous forme de routines logicielles mises en oeuvre par le microprocesseur préexistant du dispositif.

On va maintenant expliquer plus en détail la manière dont opère le dispositif de compensation du bloc 20.

Sur la figure 2, on a représenté la courbe de réponse (diagramme de Bode) du bloc 12, dont la caractéristique 24 présente une partie plate entre deux fréquences f₁ et f₂, avec une atténuation au-delà de f₂ (fonction passe-bas) et également une atténuation en-deçà de f₁ (fonction passe-haut).

Le choix de la fréquence f₁ est un compromis principalement fonction de considérations de taille des composants dans le bloc 12, mais il n'est pas critique. On notera cependant que cette fréquence f₁, qui est typiquement de 4 Hz, est notablement supérieure aux fréquences habituelles de coupure des filtres passe-haut des dispositifs classiques, de l'ordre de 1 Hz, ce qui permet de réduire la taille des composants.

La fréquence f₂, qui n'est en fait pas concernée par l'invention, est typiquement de l'ordre de 80 Hz, et définit la borne supérieure de la fenêtre d'écoute en entrée.

Le filtre passe-haut du bloc 12 est généralement un filtre du premier ordre, donnant pour la partie 30 de la caractéristique une atténuation de -6 dB/octave (on verra plus bas que l'on peut généraliser à des ordres supérieurs).

Sur la figure 3, on a reproduit en 24 cette caractéristique de la figure 2, à laquelle on va appliquer des compensations correspondant aux caractéristiques 32 et 38, pour donner la bande passante résultante 44 de la figure 4.

Pour compenser l'atténuation 30 avec le point de coupure à 4 Hz, on applique une compensation 32 avec une réponse plate 34 au-delà de f₁ = 4 Hz, et un gain 36 en-deçà. Idéalement, les deux fréquences sont identiques et égales à f₁, mais en pratique on cherche à les apparier le mieux possible afin de ne pas générer de distorsions dans le signal reconstitué. On peut utiliser une fonction amplificatrice du premier ordre, donnant une pente de +6 dB/octave. La combinaison des pentes 30 et 36 donne, en-deçà de f₁, une réponse plate, permettant donc de restituer les très basses fréquences du signal endocavitaire.

On a ainsi reconstitué les composantes spectrales qui avaient été atténuées par le filtre passe-haut d'entrée du bloc 12.

La compensation 32 conduit cependant à une amplification infinie pour une fréquence nulle, si bien qu'elle est éminemment instable. Pour corriger cette instabilité, on surajoute, dans l'étage de compensation 20, un filtre passe-haut dont la caractéristique 38 présente une réponse plate 40 au-delà d'une fréquence f₀, par exemple f₀ = 0,1 Hz, et un affaiblissement progressif 42 en-deçà de cette fréquence.

Ce second filtre présente une fonction de stabilisation de la compensation pour les très basses fréquences (inférieures à 0,1 Hz). Ce peut être un filtre du premier ordre (pente de -6 dB/octave) mais dans ce cas la résultante des caractéristiques 32 et 38 amplifierait également la composante continue du signal, notamment la tension de décalage (offset) résiduelle en sortie du bloc 12, avant numérisation par le bloc 13. C'est pourquoi, pour éviter d'avoir du gain sur la composante continue, on utilise de préférence un filtre 38 du second ordre : le premier ordre pour stabiliser la caractéristique de compensation 32 dans les très basses fréquences, et le second ordre pour annuler l'offset résiduel du bloc 12.

Dans le cas d'un filtre du second ordre, les deux pôles de celui-ci peuvent être confondus (comme dans le cas illustré, où l'on a une pente constante de -12 dB/octave en-deçà de f₀) ou distincte, les deux pôles ayant, comme on l'a indiqué, des rôles distincts.

La solution peut être généralisée à des filtres d'ordre supérieur : pour une caractéristique 30 de *n* x - 6 db/octave (filtre passe-haut d'ordre *n*), la caractéristique 36 présentera une pente de *n* x +6 dB/octave (filtre de compensation d'ordre n) et la caractéristique 42 une pente de (*n+*1) x -6 dB/octave (filtre passe-haut d'ordre *n*+1) ou, plus généralement, une pente de (*n*+*k*) x -6 dB/octave, avec *k* ≥ 1 (filtre passe-haut d'ordre *n+k*).

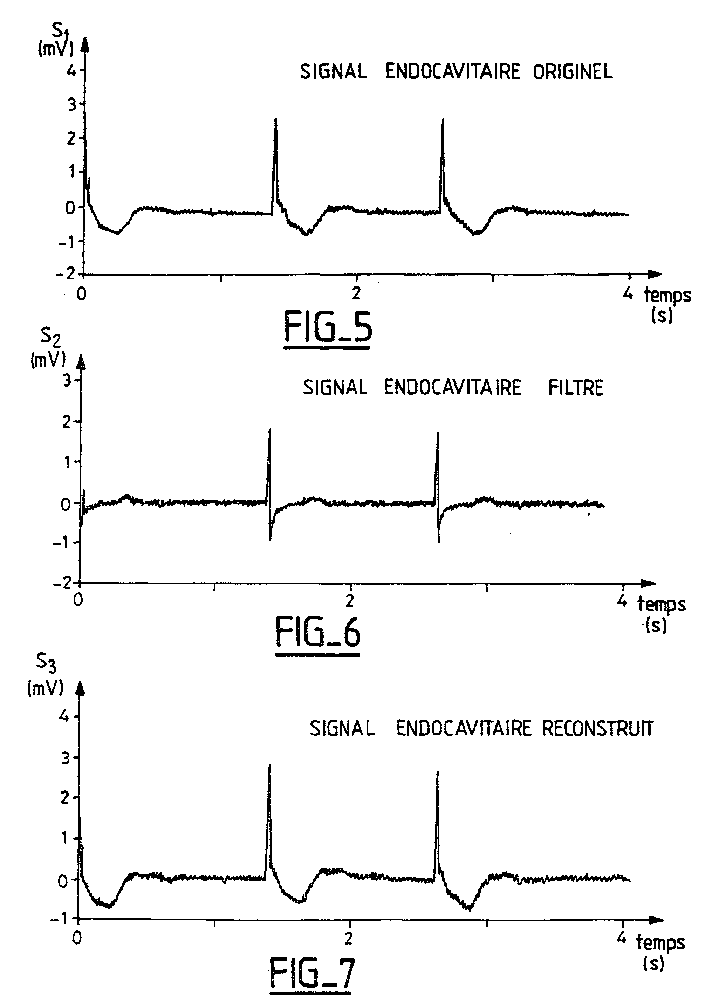

Les figures 5 à 7 illustrent l'efficacité du dispositif de l'invention sur un exemple de signal ECG.

La figure 5 correspond au signal S₁ sur le cathéter avant application du filtrage passe-haut du bloc 12.

La figure 6 représente ce même signal après application du filtrage passe-haut du bloc 12, pour un filtre passe-haut du premier ordre à fréquence de coupure de 10 Hz, c'est-à-dire le signal S₂ : on voit notamment que le segment ST (après le pic principal QRS) est notablement déformé et ne peut être analysé convenablement sur la base de ce signal.

La figure 7 illustre le signal S₃, après application de la compensation selon l'invention : si l'on compare S₃ et S₁, on voit que le signal reconstruit est extrêmement proche du signal originel, et peut donc être analysé dans tous ses aspects par le praticien ou par un logiciel d'analyse approprié.

L'invention, comme on l'a expliqué plus haut, est également applicable à l'élargissement de la bande spectrale dans le domaine des fréquences élevées.

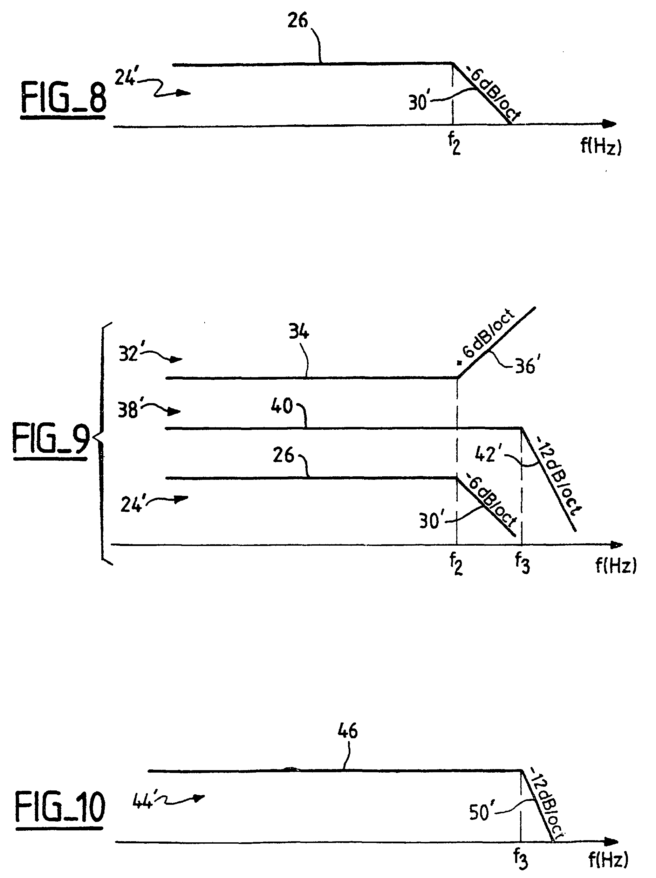

Sur les figures 8, 9 et 10, on a représenté des caractéristiques 24', 32', 38' et 44', respectivement symétriques des caractéristiques 24, 32, 38 et 44 des figures 2, 3 et 4, considérées dans le domaine des fréquences élevées du diagramme de Bode.

Au-delà d'une fréquence f₂, par exemple f₂ = 80 Hz, la partie plate 26 de la caractéristique fréquentielle 24' de l'étage d'entrée (figure 8) présente une atténuation 30' de -6 dB/octave ou, de façon plus générale, de *n* x -6 dB/octave, *n* étant l'ordre du filtre passe-bas correspondant.

La figure 9 représente, dans le haut du spectre, la caractéristique 32' de l'étage d'accentuation, qui présente en deçà de f₂ une partie plate 34 et au-delà de f₂ une accentuation 36' de +6 dB/octave, ou, dans le cas général, de *n* x +6 dB/octave.

Le second filtre passe-bas présente une caractéristique 38' comportant une partie plate 40 et une atténuation 42' au-delà d'une fréquence f₃, choisie supérieure à f₂ (par exemple une fréquence f₃ = 200 Hz). La pente de cette atténuation 42' est de -12 dB/octave ou, dans le cas général, de (*n*+1) x -6 dB/octave ou, encore plus généralement, une pente de (*n*+*k*) x -6 dB/octave, avec *k* ≥ 1 (filtre passe-haut d'ordre *n*+*k*). Ceci permet d'assurer, par analogie à ce qui a été exposé plus haut et en transposant le raisonnement aux hautes fréquences, la stabilité du système et la non-amplification du bruit résiduel en sortie du bloc 12 au-delà de la fréquence de coupure f₃.

La figure 10 illustre la bande passante résultante 44' dans le domaine des fréquences élevées, avec une partie plate 46 jusqu'à la fréquence f₃ (typiquement, f₃ = 200 Hz), permettant de restituer les composantes spectrales significatives situées notamment dans la bande 80-200 Hz, puis une forte atténuation 50' au-delà de f₃, permettant d'atténuer les composantes non significatives du signal, et tout particulièrement la composante de bruit.

## Revendications

1. Un dispositif de filtrage, recevant en entrée des signaux (S₁) de moyens de recueil de données physiologiques, et délivrant en sortie, à des moyens de traitement de ces données, des signaux (S₃) s'étendant, dans le domaine fréquentiel, sur une bande spectrale élargie, les moyens de recueil comprenant un premier filtre passe-haut (12) réduisant l'étendue de la bande spectrale du signal (S₁) reçu en entrée,
dispositif **caractérisé en ce qu'**il comprend un étage de compensation (20) comportant un dispositif d'accentuation de caractéristique fréquentielle (32) inversée par rapport à celle (24) du premier filtre passe-haut,
la fréquence de coupure (f₁) du premier filtre passe-haut étant choisie supérieure à la fréquence inférieure (f₀) de la bande spectrale élargie.

2. Le dispositif de la revendication 1, comprenant en outre un second filtre passe-haut, dont la caractéristique (38) présente une fréquence de coupure correspondant à la fréquence inférieure (f₀) de la bande spectrale élargie.

3. Le dispositif de la revendication 1, dans lequel le dispositif d'accentuation et le premier filtre passe-haut ont même fréquence de coupure (f₁) et des fonctions de transfert inverses.

4. Le dispositif de la revendication 2, dans lequel l'ordre de la fonction de transfert du second filtre passe-haut est supérieur à l'ordre de la fonction de transfert du premier filtre passe-haut.

5. Le dispositif de la revendication 1, dans lequel la fréquence inférieure (f₀) de la bande spectrale élargie est de l'ordre de 0,1 Hz.

6. Le dispositif de la revendication 1, dans lequel la fréquence de coupure (f₁) du premier filtre passe-haut est de l'ordre de 4 Hz.

7. Un dispositif de filtrage, recevant en entrée des signaux de moyens de recueil de données physiologiques, et délivrant en sortie, à des moyens de traitement de ces données, des signaux s'étendant, dans le domaine fréquentiel, sur une bande spectrale élargie, les moyens de recueil comprenant un premier filtre passe-bas réduisant l'étendue de la bande spectrale du signal (S₁) reçu en entrée,
dispositif **caractérisé en ce qu'**il comprend un étage de compensation comportant un dispositif d'accentuation de caractéristique fréquentielle (32') inversée par rapport à celle (24') du premier filtre passe-bas,
la fréquence de coupure (f₂) du premier filtre passe-bas étant choisie inférieure à la fréquence inférieure (f₃) de la bande spectrale élargie.

8. Le dispositif de la revendication 7, comprenant en outre un second filtre passe-bas, dont la caractéristique (38') présente une fréquence de coupure correspondant à la fréquence supérieure (f₃) de la bande spectrale élargie.

9. Le dispositif de la revendication 7, dans lequel le dispositif d'accentuation et le premier filtre passe-bas ont même fréquence de coupure (f₂) et des fonctions de transfert inverses.

10. Le dispositif de la revendication 8, dans lequel l'ordre de la fonction de transfert du second filtre passe-bas est supérieur à l'ordre de la fonction de transfert du premier filtre passe-bas.

11. Le dispositif de l'une des revendications 1 à 10, incorporé à un dispositif médical implantable actif.

12. Le dispositif de l'une des revendications 1 à 10, incorporé à un programmateur externe prévu pour coopérer avec un dispositif médical actif implanté.

13. Le dispositif de l'une des revendications 1 à 10, incorporé à un boîtier d'interfaçage à un programmateur externe prévu pour coopérer avec un dispositif médical actif implanté.

## Patentansprüche

1. Filtervorrichtung, welche am Eingang Signale (S₁) empfängt von Mitteln zum Empfang physiologischer Daten und am Ausgang, zu Mitteln zur Verarbeitung dieser Daten, Signale (S₃) liefert, welche sich, im Frequenzbereich, über ein verbreitertes Spektralband erstrecken, wobei die Mittel zum Empfang einen ersten Hochpassfilter (12) umfassen, welcher die Breite des Spektralbandes des am Eingang empfangenen Signals (S₁) reduziert,
wobei die Vorrichtung **dadurch gekennzeichnet ist, dass** sie eine Kompensationsstufe (20) umfasst, welche eine Vorrichtung zur Anhebung der Frequenzcharakteristik (32) aufweist, invertiert im Verhältnis zur derjenigen (24) des ersten Hochpassfilters,
wobei die Grenzfrequenz (f₁) des ersten Hochpassfilters höher als die untere Frequenz (f₀) des verbreiterten Spektralbandes gewählt wird.

2. Vorrichtung gemäß Anspruch 1, ferner umfassend einen zweiten Hochpassfilter, dessen Charakteristik (38) eine Grenzfrequenz aufweist, die der unteren Frequenz (f₀) des verbreiterten Spektralbandes entspricht.

3. Vorrichtung gemäß Anspruch 1, in welcher die Vorrichtung zur Anhebung und der ersten Hochpassfilter die gleiche Grenzfrequenz (f₁) und inverse Übertragungsfunktionen besitzen.

4. Vorrichtung gemäß Anspruch 2, in welcher die Ordnung der Übertragungsfunktion des zweiten Hochpassfilters höher ist als die Ordnung der Übertragungsfunktion des ersten Hochpassfilters.

5. Vorrichtung gemäß Anspruch 1, in welcher die untere Frequenz (f₀) des verbreiterten Spektralbandes in der Ordnung von 0,1 Hz ist.

6. Vorrichtung gemäß Anspruch 1, in welcher die Grenzfrequenz (f₁) des ersten Hochpassfilters in der Ordnung von 4 Hz ist.

7. Filtervorrichtung, die am Eingang Signale empfängt von Mitteln zum Empfang von physiologischen Daten und am Ausgang, zu Mitteln zur Verarbeitung dieser Daten, Signale liefert, die sich, im Frequenzbereich, über ein verbreitertes Spektralband erstrecken, wobei die Mittel zum Empfang einen ersten Tiefpassfilter umfassen, welcher die Breite des Spektralbandes des am Eingang empfangenen Signals (S₁) reduziert,
wobei die Vorrichtung **dadurch gekennzeichnet ist, dass** sie eine Kompensationsstufe umfasst, welche eine Vorrichtung zur Anhebung der Frequenzcharakteristik (32') aufweist, invertiert im Verhältnis zur derjenigen (24') des ersten Tiefpassfilters,
wobei die Grenzfrequenz (f₂) des ersten Tiefpassfilters höher als die untere Frequenz (f₃) des verbreiterten Spektralbandes gewählt wird.

8. Vorrichtung gemäß Anspruch 7, ferner umfassend einen zweiten Tiefpassfilter, dessen Charakteristik (38') eine Grenzfrequenz aufweist, die der oberen Frequenz (f₃) des verbreiterten Spektralbandes entspricht.

9. Vorrichtung gemäß Anspruch 7 in welcher die Vorrichtung zur Anhebung und der ersten Tiefpassfilter die gleiche Grenzfrequenz (f₂) und inverse Übertragungsfunktionen besitzen.

10. Vorrichtung gemäß Anspruch 8, in welcher die Ordnung der Übertragungsfunktion des zweiten Tiefpassfilters höher ist als die Ordnung der Übertragungsfunktion des ersten Tiefpassfilters.

11. Vorrichtung gemäß einem der Ansprüche 1-10, eingebaut in einer aktiven implantierbaren medizinischen Vorrichtung.

12. Vorrichtung gemäß einem der Ansprüche 1-10, eingebaut in ein externes Programmiergerät, das vorgesehen ist, mit einer aktiven implantierbaren medizinischen Vorrichtung zusammenzuwirken.

13. Vorrichtung gemäß einem der Ansprüche 1 bis 10, eingebaut in einen Interface-Kasten zu einem externen Programmiergerät, das vorgesehen ist, mit einer aktiven implantierbaren medizinischen Vorrichtung zusammenzuwirken.

## Claims

1. A filtering device, receiving on an input signals (S1) issued by physiological data collecting means, and issuing on an output, to means for processing said data, signals (S3) spreading, in the frequency domain, over a broadened spectral band, the collecting means including a first high-pass filter (12) narrowing the width of the spectral band of the signal (S1) received on the input,
said device being **characterised in that** it comprises a compensation stage (20) comprising an accentuation device having a frequency characteristic (32) that is inverted as compared to the frequency characteristic (24) of the first high-pass filter,
the cut-off frequency (f1) of the first high-pass filter being selected to be greater than the lower frequency (fo) of the broadened spectral band.

2. The device of claim 1, further comprising a second high-pass filter, the characteristic (38) of which has a cut-off frequency corresponding to the lower frequency (fo) of the broadened spectral band.

3. The device of claim 1, in which the accentuation device and the first high-pass filter have the same cut-off frequency (f1) and relatively inverted transfer functions.

4. The device of claim 2, in which the order of the transfer function of the second high-pass filter is greater than the order of the transfer function of the first high-pass filter.

5. The device of claim 1, in which the lower cut-off frequency (fo) of the broadened spectral band is on the order of 0.1 Hz.

6. The device of the claim 1, in which the cut-off frequency (f1) of the first high-pass filter is on the order of 4 Hz.

7. A filtering device, receiving on an input signals issued by physiological data collecting means, and issuing on an output, to means for processing said data, signals spreading, in the frequency domain, over a broadened spectral band, the collecting means including a first low-pass filter narrowing the width of the spectral band of the signal (S1) received on the input,
said device being **characterised in that** it comprises a compensation stage comprising an accentuation device having a frequency characteristic (32') that is inverted as compared to the frequency characteristic (24') of the first low-pass filter,
the cut-off frequency (f2) of the first low-pass filter being selected to be lower than the upper frequency (f3) of the broadened spectral band.

8. The device of claim 7, further comprising a second low-pass filter, the characteristic (38') of which has a cut-off frequency corresponding to the upper frequency (f3) of the broadened spectral band.

9. The device of claim 7, in which the accentuation device and the first low-pass filter have the same cut-off frequency (f2) and relatively inverted transfer functions.

10. The device of claim 8, in which the order of the transfer function of the second low-pass filter is greater than the order of the transfer function of the first low-pass filter.

11. The device of any of claims 1 to 10, incorporated to an active implantable medical device.

12. The device of any of claims 1 to 10, incorporated to an external programmer adapted to co-operate with an active implanted medical device.

13. The device of any of claims 1 to 10, incorporated to a case for interfacing to an external programmer adapted to co-operate with an active implanted medical device.
